# DEMANDE DE BREVET EUROPEEN

(11) **EP 1 338 290 A1**
(43) Date de publication de la demande: **27.08.2003**
(21) Numéro de dépôt: 03005294.8
(22) Date de dépôt: 08.08.1995
(51) Int. Cl.: A61L 9/04, C11D 3/50

(54) **Dispositif parfumant pour le parfumage et l'assainissement d'air ambiant**

(30) Priorité: 19.08.1994 CH 256194
(62) Demande divisionnaire de: 95925983.9
(71) Demandeur: Reckitt Benckiser (UK) LIMITED, Slough, Berkshire SL1 3UH (GB)
(72) Inventeur: O'Leary, Nicholas, Datchet, Slough, Berkshire SL3 9HS (GB)
(74) Mandataire: McKnight, John Crawford

(57) **Abrégé**

Dispositif utile pour le parfumage, désodorisation ou assainissement d'air ambiant ou d'enceintes fermées comprenant un élément gélifié résultant de la réticulation entre un polymère liquide fonctionnalisé et un agent de lien réticulant en présence d'une base parfumante, désodorisante ou assainissante.

## Description

### Domaine technique

La présente invention a trait au domaine de la parfumerie et plus particulièrement elle a pour objet un dispositif parfumant pour l'assainissement d'air ambiant ou d'enceintes fermées.

### Technique antérieure

L'emploi de dispositifs divers pour l'assainissement d'air ambiant a augmenté considérablement au cours des dernières années. Des désodorisants d'air ambiant par exemple sont désormais d'un usage courant dans tout ménage où ils sont utilisés tout aussi bien pour masquer les mauvaises odeurs que pour conférer des fragrances d'ambiance. Il en va de même dans les lieux publics, les bureaux ou les voitures par exemple.

Les solutions appliquées pour réaliser de tels dispositifs sont des plus variées. A titre indicatif on peut mentionner ici les systèmes basés sur la diffusion rapide d'agents assainissants, diffusion promue par l'action de diffuseurs de type "spray", aérosols ou mécaniques. Egalement connus sont les dispositifs solides constitués par des éléments imprégnés d'ingrédients assainissants actifs, lesquels éléments sont constitués par des gels, tels les gels d'agar-agar ou de stéarate de sodium, voire par des blocs de résine synthétique ou de matériaux minéraux, le plâtre par exemple ou la silice. L'état de la technique est également riche en exemples dans lesquels les dispositifs désodorisants sont constitués par des éléments d'emballage plastique renfermant les ingrédients actifs sous forme liquide, la diffusion des vapeurs assainissantes pouvant s'effectuer au travers des parois semi-perméables polymériques. Enfin, bien connus sont également les dispositifs dans lesquels la diffusion a lieu par l'entremise d'une mèche mise en contact, par l'une de ses extrémités, avec le liquide assainissant.

A l'expérience, il apparaît qu'aucun des systèmes existants ne peut satisfaire l'ensemble des critères de fonctionnalité et d'esthétique requis pour un emploi généralisé, certains dispositifs pouvant mieux s'adapter au parfumage intermittant de petits ou grands espaces, d'autres servant au contraire à l'assainissement continu.

Parmi les systèmes simples à l'efficacité éprouvée figurent ceux basés sur la diffusion d'agents assainissants dans lesquels le support est constitué par un matériau gélifié. Il s'agit de dispositifs qui utilisent en général les propriétés gélifiantes des carragénanes ou des alginates.

### Exposé de l'invention

Nous avons maintenant découvert qu'on pouvait obtenir des gels, pour emploi en tant que support dans des dispositifs désodorisants ou assainissants, par réaction, en présence d'une base parfumante, d'une substance polymérique liquide avec un agent de lien réticulant.

Comme il apparaîtra plus clairement à la lecture des exemples spécifiques donnés par la suite, les dispositifs ainsi préparés offrent plusieurs avantages par rapport aux articles de l'art antérieur. A cet égard on peut citer le fait que le support, une fois formé, apparaît comme un matériau transparent rigide et sec, pouvant renfermer une proportion élevée, jusqu'à 90% ou plus, de base parfumante. Cette dernière caractéristique est particulièrement importante car les dispositifs de l'invention peuvent ainsi être utilisés sous forme d'objets discrets de dimension réduite mais malgré tout efficaces. C'est ainsi que par exemple de tels dispositifs peuvent trouver un emploi intéressant pour le parfumage de petits espaces clos, tels que voitures, salles de bains, cabinets de toilette entre autres. Du fait de leur transparence, ils peuvent être façonnés dans des formes variées et esthétiquement plaisantes. Ce qui plus est, leur fabrication est fort simple car, une fois homogénéisé, le mélange polymérique de réaction contenant la base parfumante se présente sous forme d'un liquide qui peut être aisément versé dans des moules de forme appropriée et variée à souhait.

Enfin, des objets solides peuvent être façonnés en réduisant quelque peu la proportion de la base parfumante- par exemple à environ 70% en poids par rapport au poids du mélange polymérique - et en ajoutant au mélange de réaction des agents de remplissage tel du plâtre, de la sciure, de la poudre métallique ou autres matériaux similaires.

Nous avons découvert que certains polymères fonctionnalisés pouvaient être réticulés in situ, en présence d'une base parfumante, pour former des gels qui présentent les caractéristiques utiles mentionnées plus haut.

La présente invention a trait à un dispositif pour le parfumage, la désodorisation ou l'assainissement d'air ambiant ou d'enceintes fermées, lequel dispositif comprend un élément gélifié résultant de la réticulation in situ d'un polymère, ou copolymère, en présence d'une base parfumante, désodorisante ou assainissante et/ou d'un agent surfactant.

Plus particulièrement, l'invention concerne un dispositif comprenant un élément gélifié résultant de la réticulation in situ entre un polymère liquide fonctionnalisé, ou copolymère, et un agent de lien réticulant en présence d'une base parfumante, désodorisante ou assainissante et/ou d'un agent surfactant.

L'invention a également pour objet un procédé pour le parfumage d'un agent surfactant, lequel procédé est caractérisé en ce qu'on effectue la réticulation in situ d'un polymère, ou copolymère, en présence d'une base surfactante active et d'une base parfumante.

Parmi les polymères fonctionnalisés, dont l'emploi est préférentiel selon l'invention, figurent les polymères miscibles aux ingrédients courants de la parfumerie, lesquels polymères possèdent un ou plusieurs groupes fonctionnels. Il en va de même pour l'agent de lien réticulant qui doit posséder un ou plusieurs groupes fonctionnels complémentaires. Le mélange de ces deux éléments, en présence de la base parfumante, produit une réaction dont l'effet résulte en la formation d'un réseau tridimensionnel renfermant la base parfumante.

Les groupes fonctionnels ainsi que les groupes fonctionnels complémentaires peuvent être respectivement des groupes dérivés d'acide carboxylique, anhydride, chlorure d'acide, amine ou alcool. De préférence on choisira des polymères liquides possédant des fonctions dérivées d'acide carboxylique, anhydride ou chlorure d'acide et des agents de lien réticulants ayant une fonction amine ou alcool, l'inverse pouvant également s'appliquer. Les groupes fonctionnels sur le polymère ou l'agent de lien réticulant peuvent être mono- ou poly-fonctionnels. Afin d'obtenir un réseau tridimensionnel il faut toutefois la présence d'au moins deux groupes fonctionnels par molécule, tant pour le polymère liquide que pour l'agent réticulant.

Par "polymère liquide fonctionnalisé" on entend ici un matériau qui est liquide à température ambiante et qui possède une viscosité inférieure à 5000 poise à 25°C, de préférence de l'ordre d'environ 250 à 1000 poise. Comme indiqué plus haut, tant le polymère liquide que l'agent réticulant doivent être solubles dans la base parfumante. Une telle solubilité peut être obtenue par dissolution directe dans la base parfumante ou par adjonction d'un solvant spécifique et approprié. En pratique, il est apparu qu'avec l'emploi de proportions élevées de base parfumante, telles que celles utilisées selon les modes préférentiels de l'invention, un tel ajout est superflu.

Le polymère peut être choisi parmi les nombreux polymères capables d'être fonctionnalisés. De préférence, on choisira une polyoléfine et plus particulièrement un polymère d'une mono- ou di-oléfine contenant, avant fonctionnalisation, au moins un ou, de préférence, plusieurs groupes vinyliques.

### Manières de réaliser l'invention

Selon un mode d'exécution préférentiel de l'invention, on utilise des polymères synthétiques dérivés du butadiène, isoprène ou chloroprène. De préférence on emploie du polybutadiène maléinisé à PM de 5'000-20'000 ou du polyisoprène maléinisé à PM de 200'000-500'000. De tels polymères sont disponibles sur le marché. Il s'agit par exemple de matériaux mentionnés dans la demande de brevet européenne publiée sous le N° 023 084, dont le contenu est ici inclus par référence. A titre d'exemple on peut citer le produit connu sous le nom de "Lithene" [origine: Revertex Limited]. Parmi les différentes qualités de Lithene disponibles, de bons résultats ont été obtenus par l'emploi de "Lithene N4-9000 10MA" [origine : Revertex Ltd.]; 9000 étant le PM du polybutadiène avant maléinisation, tandis que 10MA indique le degré de maléinisation - dans ce cas 10 parties d'anhydride maléique pour 100 parties de polybutadiène (= environ 9,1%)-.

La demande de brevet européenne citée plus haut donne également des indications quant au choix des agents de lien réticulants. Le critère discriminant dans ce choix est représenté par la solubilité d'un tel agent dans le milieu réactionnel, notamment vis-à-vis des composants de la base parfumante. Comme suggéré dans la demande citée, parmi les agents qui obéissent à un tel critère figure le dihydroxy polybutadiène. La préférence cependant est donnée à des dérivés d'amines primaires éthoxylées. Parmi ces dernières, il convient tout particulièrement d'utiliser une oléylamine possédant 2 unités d'oxyde d'éthylène par molécule. D'autres agents de lien réticulants peuvent être choisis parmi le groupe d'alkylpropyldiamines à chaîne aliphatique supérieure éthoxylée ou propoxylée (par exemple le "Dicrodamet" ; origine : Croda Chemicals Ltd.), la diéthanolamine ou encore la diéthylènetriamine.

Egalement utiles se sont révélés des agents de lien réticulants constitués par des polyoxyalkylènediamines. Plus particulièrement, l'emploi de Jeffamine D-400, Jeffamine EDR-148 et Jeffamine D-2000 se montre très avantageux (Jeffamine est une marque enregistrée de Huntsman Corp.).

Egalement utiles selon l'invention sont des cocoamines ayant 5 unités d'oxyde d'éthylène par molécule. Il s'agit de produits commerciaux connus sous le nom de "Crodamet" [origine : Croda Chemicals Ltd].

Le polymère liquide fonctionnalisé et l'agent de lien réticulant sont mélangés dans un rapport molaire d'une valeur comprise entre environ 3:1 et 0,5:1, de préférence de 1:1, basé sur le rapport molaire des groupes fonctionnels présents.

L'élément gélifié selon l'invention peut également résulter du mélange de deux polymères possédant des fonctionnalités complémentaires. Bien entendu, les deux polymères doivent être solubles dans la base parfumante ou assainissante choisie. A titre d'exemple, on peut citer à cet effet le polybutadiène à fonction hydroxylée, tel le HFPB (origine: Revertex Ltd) qui gélifie en mélange avec le polybutadiène maléinisé. Parfois, l'emploi de catalyseurs spécifiques permettent un meilleur contrôle de la gélification et, à cet effet, on utilise des amines tertiaires (ex. : DAMA 1010 ; origine : Albemarle SA). Des mélange de Hycar CTBN 1300x21 (origine : B.F. Goodrich) et polybutadiène maléinisé conviennent également parfaitement.

Selon une variante du dispositif de l'invention, à titre de polymère on peut employer, au lieu d'un polymère liquide fonctionnalisé, un copolymère. Parmi les copolymères utilisés, il convient de citer particulièrement l'EMA, ou copolymère d'éthylène et anhydride maléique, substance qui se présente sous forme d'une poudre homogène fluide.

A titre de base parfumante on utilisera dans le dispositif de l'invention toute composition d'usage courant en parfumerie. Il s'agit dans ce cas de substances chimiques à l'état isolé plus fréquemment cependant de mélanges plus ou moins complexes d'ingrédients liquides volatils soit d'origine synthétique que naturelle. La nature de ces ingrédients peut être trouvée dans les ouvrages spécialisés de la parfumerie, par exemple S. Arctander (Perfume and Flavor Chemicals, Montclair N.J., USA, 1969).

Comme indiqué plus haut, un des éléments caractérisant le polymère liquide fonctionnalisé ainsi que l'agent de lien réticulant, utiles pour la préparation du dispositif de l'invention, est leur solubilité dans la base parfumante, désodorisante ou assainissante choisie. Il est entendu que tant le polymère liquide que l'agent de lien réticulant peuvent être, avant réticulation, dissous dans des solvants organiques appropriés. Toutefois une telle opération se révèle superflue dans la plupart des cas, la base active, particulièrement la base parfumante, étant suffisante pour les dissoudre rendant par conséquent inutile l'ajout d'un solvant supplémentaire.

Quoique dans ce qui précède, il a été fait mention plus particulièrement de l'effet parfumant exercé par les dispositifs selon l'invention, les mêmes principes s'appliquent à la fabrication de dispositifs analogues destinés à diffuser des vapeurs désodorisantes ou assainissantes, la base parfumante étant dans ce cas remplacée par une base désodorisante, bactéricide, insecticide, répellante ou encore attractante. Par le terme "assainissant" nous nous référons en effet ici non seulement à des substances pouvant augmenter le degré de plaisance de l'air environnant l'observateur, mais également à des substances pouvant exercer un effet attractant ou répellant vis-à-vis de certaines espèces d'insectes, par exemple vis-à-vis de la mouche domestique ou des moustiques, ou encore bactéricide.

Un des objets de la présente invention consiste donc en un dispositif parfumant, désodorisant ou assainissant comprenant un élément gélifié résultant de la réticulation in situ entre un polymère liquide fonctionnalisé, ou un copolymère, et un agent de lien réticulant en présence d'une base pavfumante, désodorisante ou assainissante.

L'invention est illustrée de manière plus détaillée par les exemples suivants dans lesquels les abréviations ont le sens usuel dans l'art et les températures sont indiquées en degrés centigrades.

### Exemple 1

Dans un récipient approprié on a placé 2,23 g de Lithene N4-9000 10MA auxquels on a ajouté 10,28 g d'une base pavfumante (Splash 115.032 BGE ; origine: Firmenich SA, Genève, Suisse) et le mélange a été brassé manuellement. Puis on a ajouté sous agitation 0,34 g de Crodamet 02. Après environ 10 min à température ambiante l'huile polymérique s'était gélifiée renfermant la base parfumante. La gélification se complète au bout d'une demi heure.

### Exemple 2

2,56 g de Lithene N4-9000 10MA ont été brassés manuellement dans un récipient approprié avec 12,43 g d'une base parfumante (Splash 115.032 BGE ; origine : Firmenich SA, Genève, Suisse), puis 0,55 g de Crodamet C5 ont été ajoutés sous agitation. Après environ 2 heures à température ordinaire, l'huile s'était gélifiée formant un bloc rigide sec. La rigidité du produit cependant n'était pas aussi prononcée que celle du produit résultant décrit dans l'exemple 1 et ce même après 24 heures.

### Exemple 3

2,29 g de Lithene N4-9000 10MA ont été brassés manuellement dans un récipient approprié avec 14,95 g d'une base parfumante (Splash 115.032 BGE ; origine : Firmenich SA, Genève, Suisse), puis 1,98 g de Crodamet 02 ont été ajoutés sous agitation. Après environ 20 min à température ambiante le mélange huileux se gélifie. La prise en masse se complète au bout de 50 min. Le produit résultant était plus mou que celui préparé à l'exemple 2 et dont le contenu en base parfumante était de 80% en poids.

### Exemple 4

1,44g de Lithene N4-9000 10MA ont été brassés manuellement dans un becher avec 14,93 g d'une base parfumante (Splash 115.032 BGE ; origine : Firmenich SA, Genève, Suisse), puis 0,22 g de Crodamet 02 ont été ajoutés sous agitation. Après 40 min le mélange se gélifie. La prise en masse se complète au bout de 3 heures. Le produit résultant, qui contient 90% en poids de base parfumante, était plus mou que celui obtenu conformément aux exemples 2 et 3, contenant respectivement 80 et 85% en poids de base parfumante.

### Exemple 5

2,50 g de Lithene N4-9000 10MA ont été brassés manuellement dans un récipient approprié avec 11,60 g d'une base parfumante (Brissago 144.034 ; origine : Firmenich SA, Genève, Suisse), puis 0,4 g de Crodamet 02 ont été ajoutés sous brassage manuel. Le mélange huileux résultant est ensuite versé dans des moules de type Barex. Après 15 min. à température ambiante l'huile s'est solidifiée en une masse gélifiée caoutchouteuse sèche.

### Exemples 6 - 8

En procédant comme indiqué à l'exemple précédent mais en remplaçant la base parfumante Brissago par des proportions identiques de respectivement Centifoline 144.036, Citronia 144.037 et Tristan 431.756 (origine : Firmenich SA, Genève, Suisse) on a obtenu des gels parfumants de bonne qualité après un temps de gélification compris entre 10 et 25 minutes.

### Exemple 9

2,54 g de Lithene N4-9000 10MA ont été mélangés avec 6,23 g d'une base parfumante (Terminator 109365B ; origine : Firmenich SA, Genève, Suisse) et le mélange a été brassé manuellement. 0,13 g de Crodamet 02 (rapport Lithene/Crodamet 3:1 environ) ont été ajoutés sous brassage. L'huile résultante s'est solidifiée sous forme de gel au bout de 15 min à température ambiante.

### Exemple 10

En opérant comme indiqué à l'exemple précédent mais en utilisant un rapport molaire de Lithene/Crodamet de 5:1 au lieu de 3:1 comme indiqué à l'exemple précédent, on a obtenu un gel collant dont les caractéristiques indiquaient un certain manque de rigidité.

### Exemple 11

1,87 g de Lithene N4-9000 10MA ont été mélangés avec 5,69 g d'une base parfumante (Terminator 109365B ; origine : Firmenich SA, Genève, Suisse) puis 0,57 g de Crodamet 02 y ont été ajoutés avec brassage manuel. Après 20 min environ à température ambiante le mélange huileux s'est solidifié sous forme de gel.

### Exemple 12

2 G environ de Lithene N4-9000 10MA ont été versés dans un récipient approprié et mélangés avec la quantité requise de base parfumante (Honeysuckle 150061 ; origine: Firmenich SA) jusqu'à dissolution complète. Les agents de lien réticulants ont été pré-mélangés et ajoutés en malaxant à la solution de polymère-base parfumante, puis le mélange résultant a été versé dans des moules et laissé à repos à température ambiante jusqu'à gélification complète.

| | | | | |
|---|---|---|---|---|
| % w/w Honeysuckle 150061 | 80,00 | 80,00 | 80,00 | 80,00 |
| % w/w Lithène N4/9000 10MA | 17,14 | 17,49 | 17,85 | 18,24 |
| % w/w Jeffamine D-400 | 2,86 | 2,27 | 1,66 | 1,01 |
| % w/w Jeffamine EDR-148 | - | 0,24 | 0,49 | 0,75 |
| Temps de gélification (min) | 59 | 33 | 21 | 7 |

### Exemple 13

2,13 G de Lithene N4-9000 10MA ont été versés dans un récipient approprié et mélangés avec 9,94 g de base parfumante (Beach 68536 ; origine: Firmenich SA). Après dissolution complète, on a ajouté sous agitation 0,35 g de Jeffamine D-400.

5 G du mélange (ce qui correspond à 4 g de base parfumante) ont été versés dans une poche thermoformée (28x46 mm) et laissée gélifier à température ambiante. Laissé à l'air, le dispositif perd 86% du poids original de la base parfumante en l'espace de 43 jours.

Des résultats analogues ont été obtenus en utilisant la base parfumante Fresh Bouquet 433213 (origine : Firmenich SA) employée dans le gel à raison de 80% en poids.

### Exemple 14

1,55 G de Lithene N4-9000 10MA ont été versés dans un récipient approprié et mélangés avec 3,82 g d'une base parfumante (Lavande de Provence 150060 ; origine : Firmenich SA) jusqu'à dissolution complète.

Dans un récipient séparé, on a mélangé 1,87 g de Hycar CTBN 1300x21 (origine : B. F. Goodrich) avec 4.13 g de la même base parfumante que ci-dessus jusqu'à dissolution complète, puis 5,37 g de cette solution ont été ajoutés à la solution parfumée du polymère obtenu précédemment.

Un gel sec, rigide mais trouble se forme rapidement à température ambiante.

### Exemple 15

17,14 G de Lithene N4-9000 10MA ont été ajoutés à un mélange constitué par 40 g d'un surfactant liquide et 40 g de terpènes orange et le tout a été mélangé manuellement jusqu'à dissolution complète.

2,86 G de Jeffamine D-400 ont été additionnés au mélange sous agitation. En moins de 2 min, à température ambiante, la masse se solidifie sous forme de gel.

La base surfactante employée était :
15.1 Marlipal 24/70 (origine : Hüls)
15.2 Marlipal O13/70 (origine : Hüls)
15.3 Lutensol ON70 (origine : BASF)

## Revendications

1. Dispositif pour le parfumage, la désodorisation ou l'assainissement d'air ambiant ou d'enceintes fermées, lequel dispositif comprend un élément gélifié résultant de la réticulation in situ d'un polymère, ou copolymère, en présence d'une base parfumante, désodorisante ou assainissante ou d'un agent surfactant.

2. Dispositif comprenant un élément gélifié résultant de la réticulation in situ entre un polymère liquide fonctionnalisé et un agent de lien réticulant en présence d'une base parfumante, désodorisante ou assainissante, et/ou d'un agent surfactant.

3. Dispositif selon la revendication 1, dans lequel l'élément gélifié résulte de la réticulation in situ entre une polyoléfine fonctionnalisée et un agent de lien réticulant constitué soit par une oléylamine ayant 2 unités d'oxyde d'éthylène par molécule, soit par une cocoamine ayant 5 unités d'oxyde d'éthylène par molécule.

4. Dispositif selon la revendication 2, résultant de la réticulation d'un polybutadiène ou polyisoprène maléinisé.

5. Dispositif selon la revendication 3, résultant de la réticulation de polybutadiène maléinisé et agent de lien réticulant employés dans une proportion molaire d'environ 1:1.

6. Dispositif selon l'une des revendications 1 à 4 **caractérisé en ce que** la base parfumante est présente dans une proportion comprise entre environ 70 et 90% en poids par rapport au poids du mélange constitué par le polymère liquide et l'agent réticulant.

7. Dispositif selon la revendication 1, **caractérisé en ce qu'**on utilise comme agent de lien réticulant une polyoxyalkylènediamine.

8. Dispositif selon la revendication 1, **caractérisé en ce que** la réticulation in situ s'effectue entre deux polymères ayant une fonctionnalité complémentaire.

9. Dispositif selon la revendication 8, **caractérisé en ce que** la réticulation in situ s'effectue entre un polybutadiène hydroxylé et un polybutadiène maléinisé.

10. Dispositif selon la revendication 8, **caractérisé en ce que** la réticulation in situ s'effectue en présence d'une amine tertiaire.

11. Procédé pour le parfumage, désodorisation ou assainissement d'air ambiant ou d'enceintes fermées, **caractérisé en ce qu'**on expose à l'action de l'air environnant, dans une pièce ou enceinte fermée, un dispositif selon l'une des revendications 1 à 10.

12. Procédé pour le parfumage d'un agent surfactant, **caractérisé en ce qu'**on effectue une réticulation in situ d'un polymère, ou copolymère, avec un agent de réticulation, en présence d'une base surfactante active et d'une base parfumante.

13. Article surfactant de nettoyage à action parfumante résultant du procédé selon la revendication 12.
